# FASCICULE DE BREVET EUROPEEN

(11) **EP 1 088 566 B1**
(45) Date de publication et mention de la délivrance du brevet: **07.07.2004**
(21) Numéro de dépôt: 99870195.7
(22) Date de dépôt: 29.09.1999
(51) Int. Cl.: A61M 5/00, B65D 5/42, B01L 9/06

(54) **Dispositif de manutention d'objets permettant le passage aisé d'un fluide de traitement**
Vorrichtung zum Handhaben von Objekten und Ermöglichen einer ungehinderten Durchströmung eines Behandlungsfluids
Object handling device allowing easy passage of treatment fluid

(43) Date de publication de la demande: 04.04.2001
(73) Titulaire: Sybermat, 1348 Louvain-la-Neuve (BE)
(72) Inventeur: Cousin, Gery, 1640 Rhode-Saint-Genèse (BE)
(74) Mandataire: Claeys, Pierre

(56) Documents cités:
- EP-A- 0 790 063
- WO-A-94/14484
- DE-A- 4 419 291
- US-A- 2 902 169
- US-A- 4 142 633
- US-A- 5 169 603
- US-A- 5 462 220
- US-A- 5 482 067

## Description

La présente invention concerne un dispositif de manutention destiné à recevoir des objets qui doivent subir un traitement. Un tel dispositif selon le préambule de la revendication 1 est connu par exemple de US-A-5 482 067. Il peut s'agir en particulier d'un dispositif pour transporter des seringues à traiter, avant leur remplissage, par exemple à la vapeur ou avec des produits de stérilisation et pour les amener ensuite à un poste de remplissage.

Il y a un besoin constant d'améliorer la manière de travailler dans de telles circonstances afin de rendre plus sûrs les résultats de tels traitements et de réduire le plus possible des manipulations longues et coûteuses de préparation et de rangement des objets en vue de leur traitement et entre autre de leur remplissage. L'invention a pour but de proposer une amélioration sensible de ce genre de travail.

A cet effet, le dispositif suivant l'invention comporte les caractéristiques de la revendication 1. Il comporte une boîte qui peut comprendre un corps de boîte à faces longitudinales et latérales, un couvercle de fermeture du corps de boîte, et des moyens de fixation des objets dans la boîte, agencés pour y bloquer ces objets en ordre et position par fermeture du couvercle.

Le corps de boîte et le couvercle proposés présentent de plus de nombreuses ouvertures en vue d'un passage aisé d'un fluide de l'extérieur vers l'intérieur de la boîte et vice versa.

Suivant une forme de réalisation de l'invention, le corps de boîte est formé à partir d'une tôle d'une pièce, découpée et pliée de manière à présenter une plaque de base rectangulaire et, rattachées à celle-ci par des plis, deux faces latérales de boîte et deux faces longitudinales de boîte.

Dans une forme particulière de l'invention, la plaque de base comporte des rainures traversantes, parallèles aux plis de jonction des faces latérales, qui débuterit à distance du pli de jonction à une face longitudinale et qui s'étendent au delà du pli de jonction à l'autre face longitudinale, à travers cette 'autre face jusqu'à un distance choisie du bord de cette face qui est opposé au pli de jonction.

Dans une variante de l'invention, les faces latérales du couvercle comportent chacune au moins une ouverture oblongue parallèle au pli de jonction et de préférence en regard d'une ouverture oblongue, ménagée dans la face latérale correspondante du corps de boîte, lorsque le corps de boîte est fermé par le couvercle.

D'autres détails et particularités de l'invention ressortiront des revendications secondaires et de la description des dessins qui sont annexés au présent mémoire et qui illustrent, à titre d'exemples non limitatifs, une forme de réalisation particulière du dispositif suivant l'invention.

La figure 1 montre une vue du dessus d'un corps de boîte de l'invention.

La figure 2 est une vue de côté du corps de boîte de la figure 1.

Les figures 3 et 4 montrent respectivement une face longitudinale antérieure et, vue depuis celle-ci suivant la ligne IV-IV de la figure 1, une face postérieure du même corps de boîte.

La figure 5 montre une vue du dessus d'un couvercle de l'invention.

La figure 6 est une vue de côté du couvercle de la figure 5.

La figure 7 montre une face longitudinale antérieure du même couvercle.

La figure 8 est une vue en plan d'un peigne de guidage des objets dans le dispositif de l'invention.

La figure 9 est une vue en élévation d'un objet concerné par l'invention, un corps de seringue dans le cas de l'exemple considéré.

La figure 10 est une vue de côté d'une boîte, en position ouverte, du dispositif de l'invention.

Dans les différentes figures, les mêmes notations de référence désignent des éléments identiques ou analogues.

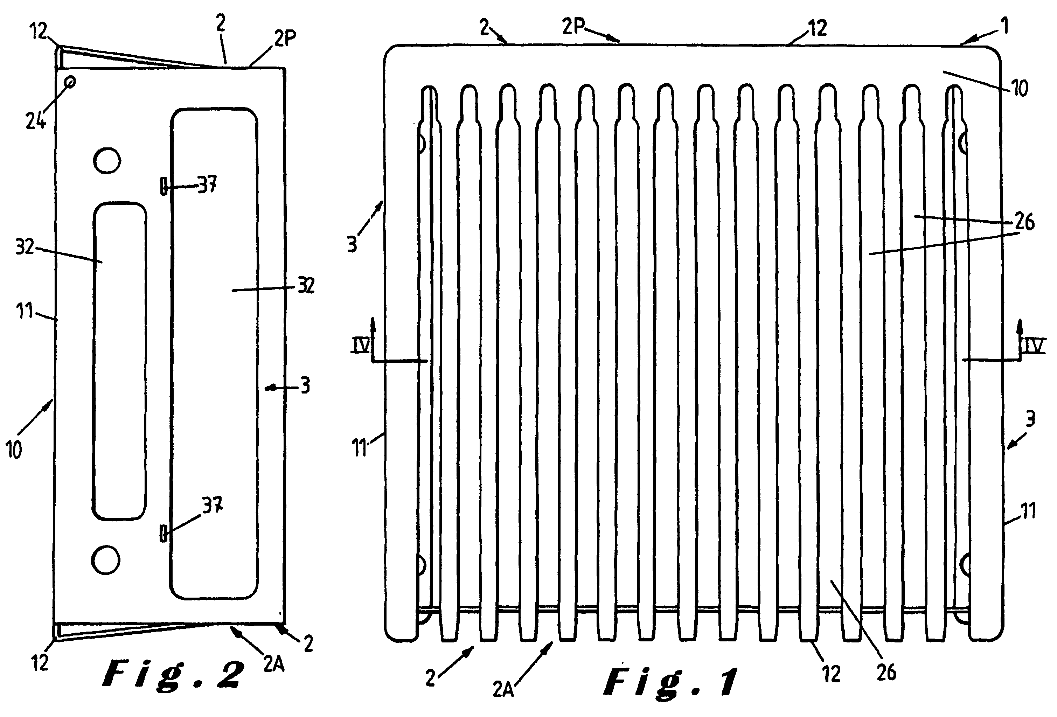

Le dispositif de l'invention comprend une boîte comportant (figures 1 à 7) un corps de boîte 1 à faces longitudinales 2 et latérales 3, un couvercle 4 de fermeture du corps de boîte 1, et des moyens de fixation des objets dans la boîte, agencés pour les y bloquer en ordre et position par fermeture du couvercle 4.

Le corps de boîte 1 et le couvercle 4 présentent de nombreuses ouvertures en vue d'un passage aisé d'un fluide de traitement de l'extérieur vers l'intérieur de la boîte et vice versa.

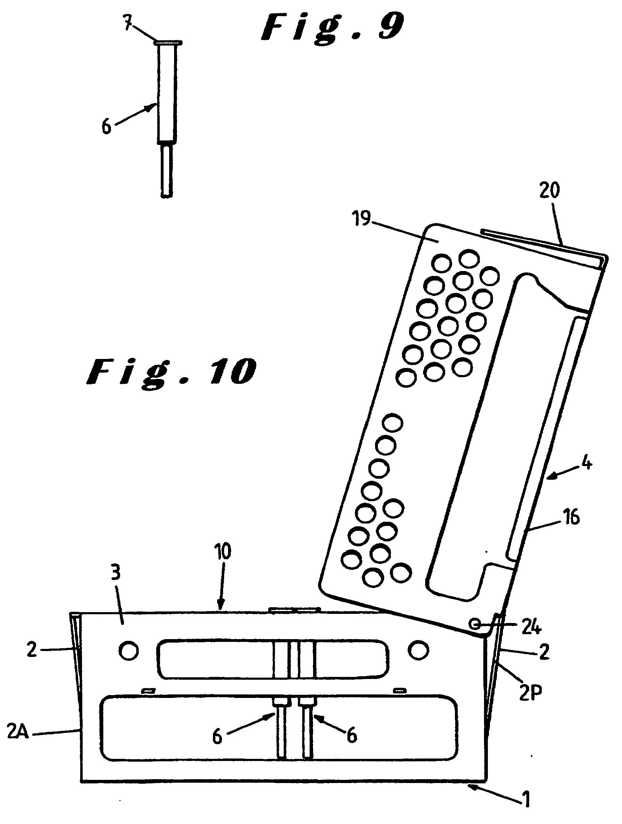

Les dessins des figures correspondant au cas où les objets sont (figure 9) des corps de seringue 6 munis d'un collet 7 à une extrémité, la description ci-après est faite dans ce contexte.

Le corps de boîte 1 (figures 1 à 4) est avantageusement formé à partir d'une tôle d'une pièce, découpée et pliée de manière à présenter une plaque de base 10 rectangulaire et, rattachées à celle-ci par des plis 11 et respectivement 12, deux faces latérales 3 de boîte et deux faces longitudinales 2 de boîte.

En particulier pour le cas des corps de seringue 6, la plaque de base 10 et les faces latérales 3 et longitudinales 2 sont disposées de manière à ce que, dans une position de remplissage de la boîte, les faces latérales 3 et longitudinales 2 s'étendent sous la plaque de base 10.

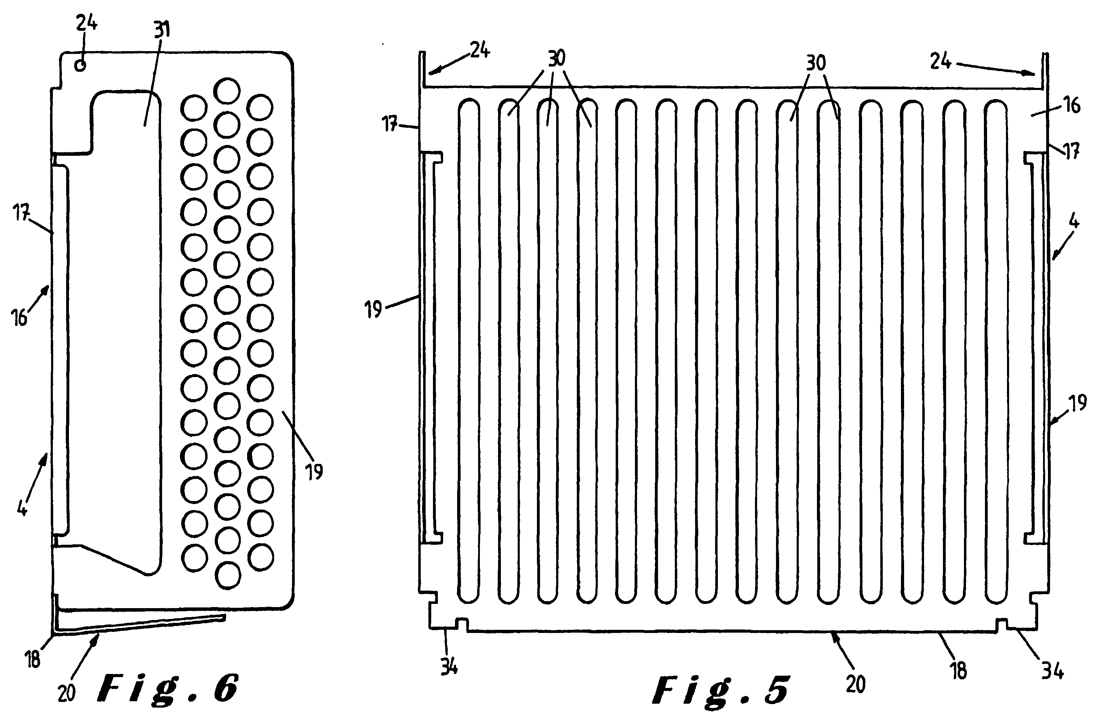

Le couvercle 4 (figures 5 à 7) est aussi formé de préférence à partir d'une tôle d'une pièce, découpée et pliée de manière à présenter une plaque de recouvrement 16 rectangulaire pour le corps de boîte 1 et, rattachées à cette plaque 16 par des plis 17 et respectivement 18, deux faces latérales 19 de couvercle et au moins une face longitudinale 20 de couvercle.

Les dimensions des faces latérales 19 sont choisies de préférence pour que, lorsque le corps de boîte 1 est fermé par le couvercle 4, ces faces latérales 19 recouvrent pratiquement le périmètre des faces latérales 3, sauf au bas de la boîte.

Les dimensions de la ou des faces longitudinales 20 du couvercle 4 sont choisies de préférence pour que, dans les mêmes conditions de boîte fermée, elles recouvrent la face longitudinale 2 associée sur au moins la moitié de la hauteur de celle-ci et sur pratiquement toute sa longueur.

Le couvercle 4 pourrait être d'un type à quatre faces, à poser sur et à détacher chaque fois du corps de boîte 1.

Cependant, pour faciliter les manipulations de la boîte, le dispositif de l'invention peut comporter (figure 10) un système de charnière 24 pour la liaison du couvercle 4 au corps de boîte 1. Dans ce cas, du côté du système à chamière 24, la face longitudinale peut être omise, comme c'est le cas dans les figures.

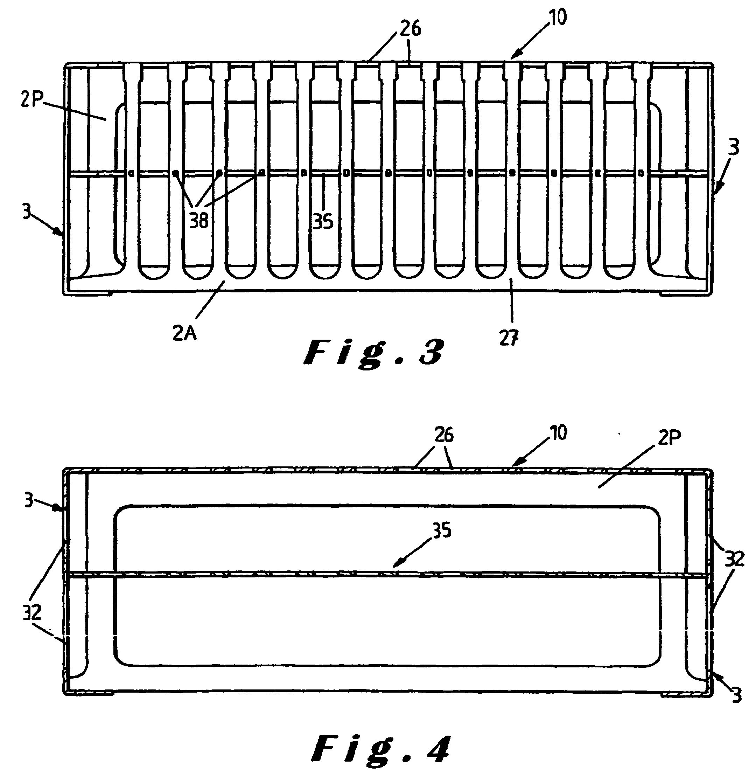

Dans le cas d'objets comme les corps de seringue 6, la plaque de base 10 comporte avantageusement (figures 1 et 3) des rainures traversantes 26, parallèles aux plis 11 de jonction des faces latérales 3 et dont la largeur correspond au diamètre du corps de seringue 6 proprement dit. Ces rainures 26 peuvent débuter à distance du pli 12 de jonction à la face longitudinale postérieure 2P et s'étendre au delà du pli 12 de jonction à la face longitudinale antérieure 2A, à travers cette face jusqu'à un distance choisie du bord 27 de cette face 2P qui est opposé au pli de jonction 12.

Les dimensions et forme de la boîte sont avantageusement choisies pour que la longueur de chaque rainure 26 dans la plaque de base 10 permette d'y installer un nombre entier de corps de seringue 6. De même, l'écart entre deux rainures 26 est choisi entre autre de manière à ce que les collets 7 des corps de seringue 6 disposés dans deux rainures 26 contiguës puissent librement poser sur ladite plaque de base 10.

Lorsque le couvercle 4 monté à charnière est en position ouverte, les corps de seringue 6 peuvent être glissés (figure 1) l'un derrière l'autre dans chaque rainure 26 de la plaque de base 10, et être maintenus en appui sur celle-ci par leur collet 7 pendant le temps d'un chargement de la boîte. L'introduction des objets 6 dans les rainures 26 est facilité par le fait que, comme le montrent les figures 1, 3 et 10, chaque rainure 26 est ouverte à son extrémité opposée au système de charnière 24. Cette ouverture des rainures 26 est encore plus intéressante pour retirer ultérieurement les corps de seringue 6 de la boîte, par exemple à l'endroit d'une machine de remplissage de ceux-ci, puisqu'ils pourront être directement glissés en bon ordre hors des rainures 26 et amenés ainsi sur des guides appropriés de cette machine.

Le couvercle 4 est monté sur le corps de boîte 1 de manière à ce qu'en position fermée, sa plaque de recouvrement 16 maintienne les collets 7 suffisamment près de, sinon contre, la plaque de base 10.

Pour le traitement précité, le couvercle 4 peut présenter lui aussi des rainures traversantes 30 qui s'étendent parallèlement aux plis 17 de jonction des faces latérales 19 et qui, lorsque le couvercle 4 est en position de fermeture de la boîte, sont disposées en regard des rainures 26 de la plaque de base 1.

Il est avantageux (figures 2, 3 et 10) que les faces latérales 19 du couvercle 4 comportent chacune au moins une ouverture 31 oblongue ou semblable, parallèle à son pli de jonction 17 et de préférence en regard d'une ouverture 32 oblongue ou semblable ménagée dans la face latérale 3 correspondante du corps de boîte 1, lorsque le corps de boîte 1 est fermé par le couvercle 4. Ceci a pour effet qu'en saisissant par un de ses côtés latéraux la boîte fermée, on passe automatiquement un ou des doigts à travers ces ouvertures 31 et 32 et l'on assure ainsi un blocage du couvercle 4 sur le corps de boîte 1.

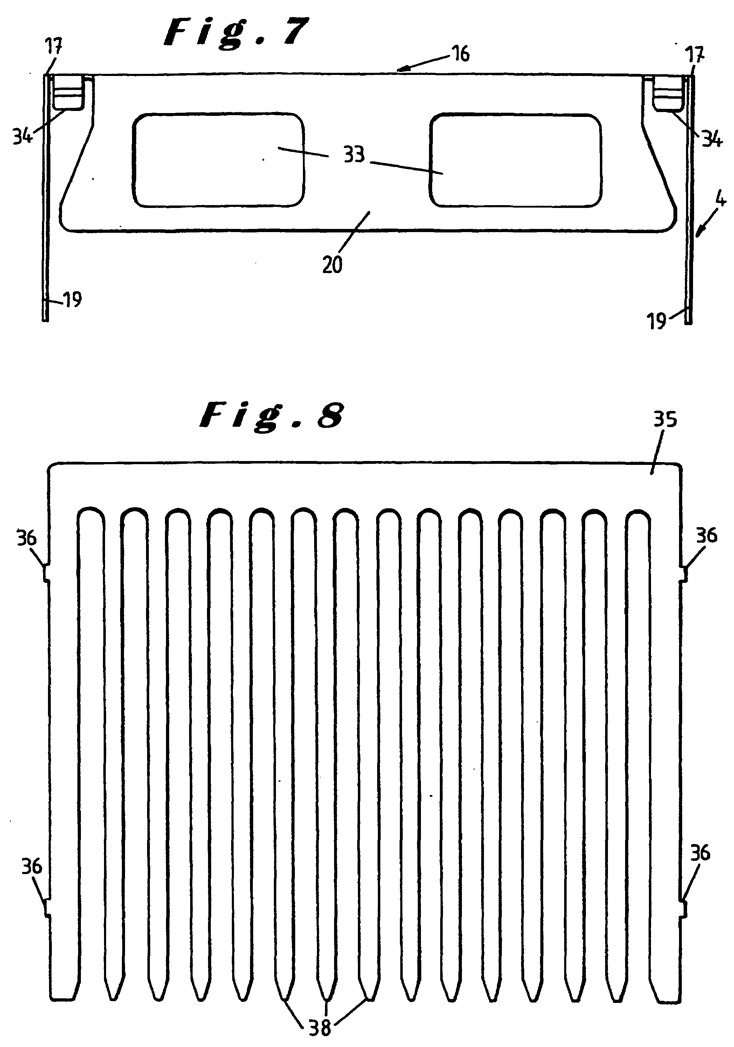

A cet effet également (figure 7), la ou, le cas échéant, les faces longitudinales 20 du couvercle 4 peuvent être percées d'au moins une à quatre grandes ouvertures 33 dont la surface totale ou les surfaces cumulées peuvent être de l'ordre de la moitié de la surface de la face longitudinale 20 considérée. Ainsi, lorsqu'on saisit par cette face 20 la boîte fermée, on passe automatiquement un ou des doigts à travers la ou les ouvertures 33 et l'on prend appui contre la face longitudinale 2 correspondante (figure 3) et cela bloque aussi le couvercle 4 en position fermée.

Des moyens, par exemple deux languettes élastiques 34 prévues sur le couvercle 4, peuvent servir à assurer un autre blocage en position fermée de celui-ci sur le corps de boîte 1.

On notera aux figures 2, 6 et 10 que les faces longitudinales 2 et 20 forment avec la plaque de base 10 ou de recouvrement 16 un angle différent de 90°. Cela n'est cependant pas une obligation.

Dans le cas des corps de seringue 6, une boîte du genre décrit ci-dessus peut avoir des dimensions de l'ordre de 90 mm de haut, 250 mm de long et 230 mm de large.

Il doit être entendu que l'invention n'est nullement limitée aux formes de réalisation décrites et que bien des modifications peuvent être apportées à ces dernières sans sortir du cadre des revendications.

Ainsi, la boîte du dispositif de l'invention peut ne pas être réalisée en tôle (métallique, de préférence inoxydable) pliée mais peut être fabriquée par injection de matière plastique appropriée, le corps de boîte 1 et le couvercle 4 étant par exemple directement reliés par un système de charnière injectée connu ou étant injectés en tant que deux pièces libres l'une de l'autre. Après une telle injection, les faces latérales et longitudinales peuvent encore devoir être mises en position par pliage, soudure, emboîtement, etc. Cependant on considère aussi dans le cadre de la présente invention une boîte dont le corps 1 et/ou le couvercle 4 sont directement produits, mis en forme, lors de l'injection.

Un peigne de guidage 35 (figure 8) peut être ajouté au corps de boîte 1, en dessous de la plaque de base 10, pour coopérer avec celle-ci en vue du bon guidage des corps de seringue 6. Dans ce cas bien sûr, les dimensions du peigne 35 correspondront à celles de la plaque de base 10 et à la place disponible à l'endroit où il sera fixé, par exemple par des languettes 36 dans des encoches 37 dans les faces latérales 3 (figure 2). De plus, les ou des extrémités 38 (figure 8) des dents du peigne 35 peuvent être fixées de différentes manières à la face longitudinale 2 (figure 10), par exemple par enfoncement dans des trous appropriés ménagés dans cette face 2.

En appliquant les dispositions décrites ci-dessus, on obtient une boîte appropriée qui conserve l'ordre et la position des objets 6 à y mettre et qui ne s'ouvre pas non intentionnellement, même si l'on secoue et/ou retourne la boîte sans ménagements, étant donné que les objets 6 y sont bien maintenus et que l'on est amené à tenir fermée la boîte qui l'est au moment où on la prend en main.

Des rainures 26 étagées sont par exemple applicables, au cas où les collets 7 présentent en plus des méplats, pour qu'un étage inférieur maintienne le corps de seringue en place et qu'un étage supérieur (normalement à ouverture plus grande correspondant à la distance entre méplats) maintienne fixe en rotation, par les méplats, le corps de seringue 6 tout au long de son séjour dans la boîte.

### Légendes

- 1: corps de boîte
- 2: face longitudinale de 1 :
- 2A: antérieure
- 2P: postérieure
- 3: face latérale de 1
- 4: couvercle
- 6: objet ou corps de seringue
- 7: collet de 6
- 10: plaque de base de 1
- 11: pli entre 10 et 3
- 12: pli entre 10 et 2
- 16: plaque de recouvrement de 4
- 17: pli entre 16 et 19
- 18: pli entre 16 et 20
- 19: face latérale de 4
- 20: face longitudinale de 4
- 24: système de charnière
- 26: rainures traversantes de 10
- 27: bord opposé de 2A
- 30: rainures traversantes de 16
- 31: ouverture dans 19
- 32: ouverture dans 3
- 33: ouverture(s) dans 20
- 34: languettes élastiques sur 4
- 35: peigne de guidage
- 36: languettes de 35
- 37: encoches dans 3
- 38: extrémités des dents de 35

## Revendications

1. Dispositif de manutention d'objets identiques, tels que corps de seringue ou seringues, destinés à subir un traitement en des position suspendue et ordre déterminés qui doivent être conservés, **caractérisé en ce qu'**il est constitué d'une boîte comportant :
- un corps de boîte (1) d'allure parallélépipédique présentant, dans sa partie supérieure, une plaque de support (10) pour des objets à suspendre,
- des rainures traversantes (26) parallèles réalisées dans la plaque de support (10) parallèlement à un de ses bords (11) et s'étendant, pour faire office de butée, à distance d'un des bords (12), et jusqu'à l'autre bord (12) qui lui est opposé,
- des rainures traversantes situées dans le prolongement des rainures (26) et réalisées dans la paroi (2A) de la boîte jusqu'à proximité de sont bord inférieur (27) afin de permettre, par glissement sur la plaque (10), l'introduction des objets à suspendre dans la boîte,
- un couvercle (4) articulé (en 24) sur le corps de boîte (1) et agencé pour, en position fermée, se placer, d'une part, sensiblement parallèlement à la plaque de support (10) susdite et à une distance telle de cette dernière qu'il puisse s'opposer au déplacement des objets suivant une direction perpendiculaire à cette plaque (10) et, d'autre part, sensiblement parallèlement à la paroi (2A) précitée et sur au moins une partie de la hauteur de cette dernière afin de s'opposer au déplacement des objets suivant une direction parallèle aux bords (11) précités,
- des moyens (34) agencés pour maintenir temporairement le couvercle (4) en position fermée,
- des ouvertures réalisées dans le corps (1) et le couvercle (4) de la boite afin de permettre une circulation aisée d'un fluide de traitement dans tout le volume intérieur de la boîte tant à partir de l'extérieur de celle-ci qu'à partir de l'intérieur.

2. Dispositif suivant la revendication 1, **caractérisé en ce que** le corps de boîte (1) est formé à partir d'une tôle d'une pièce, découpée et pliée de manière à présenter une plaque de base (10) rectangulaire et, rattachées à celle-ci par des plis (11, 12), deux faces latérales (3) de boîte et deux faces longitudinales (2) de boîte.

3. Dispositif suivant la revendication 2, **caractérisé en ce que**, par rapport à la plaque de base (10), les faces latérales (3) et longitudinales (2) sont disposées de manière à ce que, dans une position de remplissage de la boîte, elles s'étendent sous la plaque de base (10).

4. Dispositif suivant la revendication 1, **caractérisé en ce que** le couvercle (4) est formé à partir d'une tôle d'une pièce, découpée et pliée de manière à présenter une plaque de recouvrement (16) rectangulaire pour le corps de boîte (1) et, rattachées à cette plaque par des plis (17, 18), deux faces latérales (19) de couvercle et au moins une face longitudinale (20) de couvercle.

5. Dispositif suivant l'une quelconque des revendications 1 à 4, **caractérisé en ce qu'**il comporte un système de charnière (24) pour la liaison du couvercle au corps de boîte (1).

6. Dispositif suivant l'une quelconque des revendications 2 à 5, **caractérisé en ce que** la plaque de base (10) comporte des rainures traversantes (26), parallèles aux plis (11) de jonction des faces latérales (3), qui débutent à distance du pli (12) de jonction à une face longitudinale (2P) et qui s'étendent au delà du pli (12) de jonction à l'autre face longitudinale (2A), à travers cette face (2A) jusqu'à une distance choisie du bord (27) de cette face (2A) qui est opposé au pli (12) de jonction.

7. Dispositif suivant la revendication 6, **caractérisé en ce que** le couvercle (4) présente des rainures traversantes (30), qui s'étendent parallèlement aux plis (17) de jonction des faces latérales (19) et qui, lorsque le couvercle (4) est en position de fermeture de la boîte, sont disposées en regard des rainures (26) de la plaque de base (10).

8. Dispositif suivant l'une quelconque des revendications 4 à 7, **caractérisé en ce que** les faces latérales (19) du couvercle (4) comportent chacune au moins une ouverture oblongue (31) parallèle au pli de jonction (17) et de préférence en regard d'une ouverture oblongue (32) ménagée dans la face latérale (3) correspondante du corps de boîte (1), lorsque le corps de boîte (1) est fermé par le couvercle (4).

9. Dispositif suivant l'une quelconque des revendications 4 à 8, **caractérisé en ce que** la ou, le cas échéant, les faces longitudinales (20) du couvercle (4) sont percées d'au moins une à quatre grandes ouvertures (33) dont la surface totale ou les surfaces cumulées sont de l'ordre de la moitié de la surface de la face longitudinale (20) considérée.

## Patentansprüche

1. Vorrichtung zum Handhaben von identischen Objekten, beispielsweise von Spritzenkörpern oder Spritzen, die in einer bestimmten hängenden Position und Reihenfolge, die es beizubehalten gilt, behandelt werden sollen, **dadurch gekennzeichnet, dass** sie aus einem Gehäuse besteht, umfassend:
- einen die Form eines Parallelepipeds aufweisenden Gehäusekörper (1), der in seinem oberen Bereich mit einer Stützplatte (10) zum Aufhängen von Objekten versehen ist,
- parallel verlaufende, durchgehende Nuten (26), die parallel zu einer der Kanten (11) der Stützplatte (10) in diese eingebracht sind und die sich, um als Anschlag dienen zu können, im Abstand zu einer der Kanten (12) und bis zur anderen, ihr gegenüberliegenden Kante (12) erstrecken,
- die Nuten (26) verlängernde, durchgehende Nuten, die in der Wand (2A) des Gehäuses bis in die Nähe seiner unteren Kante (27) eingebracht sind, so dass durch Verschieben der Platte (10) die aufzuhängenden Gegenstände in das Gehäuse eingeführt werden können,
- einen auf dem Gehäusekörper (1) bei (24) angelenkter Deckel (4), der derart angeordnet ist, dass er in geschlossener Stellung einerseits im Wesentlichen parallel zur oben genannten Stützplatte (10) und in einem Abstand zu dieser zu liegen kommt, wodurch er in die Lage versetzt wird, der Verschiebung der Gegenstände in eine senkrecht zu dieser Platte (10) verlaufende Richtung entgegenzuwirken, und andererseits im Wesentlichen parallel zur vorgenannten Wand (2A) und auf mindestens einem Teil ihrer Höhe zu liegen kommt, so dass er der Verschiebung der Objekte in eine parallel zu den vorgenannten Kanten (11) verlaufende Richtung entgegenwirken kann,
- Mittel (34), die derart angeordnet sind, dass sie vorübergehend den Deckel (4) in der geschlossenen Stellung halten können,
- in das Gehäuse (1) und den Deckel (4) des Gehäuses eingebrachte Öffnungen, so dass ein Behandlungsfluid den gesamten Innenraum des Gehäuses sowohl von außerhalb als auch von innerhalb desselben ungehindert durchströmen kann.

2. Vorrichtung nach Anspruch 1, **dadurch gekennzeichnet, dass** der Gehäusekörper (1) aus einem Blechteil gebildet ist, das derart beschnitten und gefalzt ist, dass er eine rechteckige Bodenplatte (10) und zwei Gehäuseseitenflächen (3) sowie zwei Gehäuselängsseiten (2) aufweist, die mit der Bodenplatte über Falze (11, 12) verbunden sind.

3. Vorrichtung nach Anspruch 2, **dadurch gekennzeichnet, dass** die Seitenflächen (3) und die Längsseiten (2) im Verhältnis zur Bodenplatte (10) derart angeordnet sind, dass sie sich in der Einfüllposition des Gehäuses unterhalb der Bodenplatte (10) erstrecken.

4. Vorrichtung nach Anspruch 1, **dadurch gekennzeichnet, dass** der Deckel (4) aus einem Blechteil gebi·ldet ist, das derart beschnitten und gefalzt ist, dass er eine rechteckige Abdeckplatte (16) für den Gehäusekörper (1) und zwei Deckelseitenflächen (19) sowie mindestens eine Deckellängsseite (20) aufweist, die mit der Bodenplatte über Falze (17, 18) verbunden sind.

5. Vorrichtung nach einem der Ansprüche 1 bis 4, **dadurch gekennzeichnet, dass** sie ein Scharniersystem (24) zur Verbindung des Deckels mit dem Gehäusekörper (1) umfasst.

6. Vorrichtung nach einem der Ansprüche 2 bis 5, **dadurch gekennzeichnet, dass** die Bodenplatte (10) durchgehende Nuten (26) aufweist, die parallel zu den Verbindungsfalzen (11) der Seitenflächen (3) verlaufen und im Abstand des Verbindungsfalzes (12) an einer Längsseite (2P) ihren Anfang nehmen, sich über den Verbindungsfalz (12) hinaus zur anderen Längsseite (2A) durch diese Seite (2A) hindurch erstrecken und in einem gewählten Abstand von der Kante (27) dieser dem Verbindungsfalz (12) gegenüberliegenden Seite (2A) enden.

7. Vorrichtung nach Anspruch 6, **dadurch gekennzeichnet, dass** der Deckel (4) durchgehende Nuten (30) aufweist, die parallel zu den Verbindungsfalzen (17) der Seitenflächen (19) verlaufen und bei geschlossenem Deckel (4) den Nuten (26) der Bodenplatte (10) gegenüberliegen.

8. Vorrichtung nach einem der Ansprüche 4 bis 7, **dadurch gekennzeichnet, dass** die Seitenflächen (19) des Deckels (4) jeweils mindestens eine längliche Öffnung (31) aufweisen, die parallel zum Verbindungsfalz (17) verläuft und vorzugsweise einer in der entsprechenden Seitenfläche (3) des Gehäusekörpers (1) eingebrachten länglichen Öffnung (32) gegenüberliegend angeordnet ist, wenn der Deckel (4) den Gehäusekörper (1) verschließt.

9. Vorrichtung nach einem der Ansprüche 4 bis 8, **dadurch gekennzeichnet, dass** in die Längsseite bzw. Längsseiten (20) des Deckels (4) mindestens eine und bis zu vier große Öffnungen (33) gebohrt sind, deren Gesamtfläche bzw. die Summe ihrer Gesamtflächen die Hälfte der Fläche der betreffenden Längsseite (20) beträgt, bzw. betragen.

## Claims

1. Device for handling identical objects, such as syringe bodies or syringes, intended to undergo treatment in a given suspended position and order which must be preserved, **characterised in that** it consists of a box comprising:
- a box body (1) with a parallelepipedal appearance having, in its top part, a support plate (10) for objects to be suspended,
- parallel traversing grooves (26) produced in the support plate (10) parallel to one of its edges (11) and extending, in order to serve as a stop, at a distance from one of the edges (12), and as far as the other edge (12) which is opposite thereto,
- traversing grooves situated in line with the grooves (26) and produced in the wall (2A) of the box as far as a point close to its bottom edge (27) in order to allow, by sliding on the plate (10), the introduction of objects to be suspended in the box,
- a lid (4) articulated (at 24) on the box body (1) and arranged so as, in the closed position, to be placed firstly substantially parallel to the above-mentioned support plate (10) and at such a distance from the latter that it can oppose the movement of objects in a direction perpendicular to this plate (10) and, secondly, substantially parallel to the aforementioned wall (2A) and over at least part of the height of the latter in order to oppose the movement of objects in a direction parallel to the aforementioned edges (11),
- means (34) arranged so as to temporarily hold the lid (4) in the closed position,
- openings produced in the body (1) and lid (4) of the box in order to allow easy circulation of a treatment fluid through the internal volume of the box both from the outside thereof and from the inside.

2. Device according to Claim 1, **characterised in that** the box body (1) is formed from a metal sheet in one piece, cropped and bent so as to have a rectangular base plate (10) and, attached to this by folds (11, 12), two lateral box faces (3) and two longitudinal box faces (2).

3. Device according to Claim 2, **characterised in that**, with respect to the base plate (10), the lateral (3) and longitudinal (2) faces are disposed so that, in a position of filling the box, they extend below the base plate (10).

4. Device according to Claim 1, **characterised in that** the lid (4) is formed from a metal sheet in one piece, cropped and bent so as to have a rectangular covering plate (16) for the box body (1) and, attached to this plate by folds (17, 18), two lateral lid faces (19) and at least one longitudinal lid face (20) .

5. Device according to any one of Claims 1 to 4, **characterised in that** it comprises a hinge system (24) for connecting the lid to the box body (1).

6. Device according to any one of Claims 2 to 5, **characterised in that** the base plate (10) comprises traversing grooves (26), parallel to the junction folds (11) of the lateral faces (3), which start at a distance from the junction fold (12) at a longitudinal face (2P) and which extend beyond the junction fold (12) at the other longitudinal face (2A), across this face (2A) to a chosen distance from the edge (27) of this face (2A) which is opposite to the junction fold (12).

7. Device according to Claim 6, **characterised in that** the lid (4) has through grooves (30), which extend parallel to the junction folds (17) of the lateral faces (19) and which, when the lid (4) is in the box closure position, are disposed opposite the grooves (26) in the base plate (10).

8. Device according to any one of Claims 4 to 7, **characterised in that** the lateral faces (19) of the lid (4) each comprise at least one oblong opening (31) parallel to the junction fold (17) and preferably opposite an oblong opening (32) provided in the corresponding lateral face (3) of the box body (1), when the box body (1) is closed by the lid (4).

9. Device according to any one of Claims 4 to 8, **characterised in that** the longitudinal face or where applicable faces (20) of the lid (4) have in them at least one to four large openings (33) where the total surface areas or the cumulative surface areas are around half the surface area of the longitudinal face (20) in question.
